(19) Europäisches Patentamt
European Patent Office
Office européen des brevets

(11) **EP 4 116 285 A1**

(12) **EUROPÄISCHE PATENTANMELDUNG**

(43) Veröffentlichungstag:
**11.01.2023 Patentblatt 2023/02**

(21) Anmeldenummer: **21020361.8**

(22) Anmeldetag: **08.07.2021**

(51) Internationale Patentklassifikation (IPC):
**C07C 41/01** (2006.01)  **C07C 41/38** (2006.01)
**C07C 43/04** (2006.01)  **C07C 41/42** (2006.01)
**C01B 3/48** (2006.01)

(52) Gemeinsame Patentklassifikation (CPC):
(C-Sets verfügbar)
**C01B 3/48; C07C 41/01; C07C 41/42**  (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Linde GmbH**
**82049 Pullach (DE)**

(72) Erfinder:
• **Fischer, Anna-Maria**
**82049 Pullach (DE)**
• **Peschel, Andreas**
**82049 Pullach (DE)**

(74) Vertreter: **Fischer, Werner**
**Linde GmbH**
**Intellectual Property EMEA**
**Dr.-Carl-von-Linde-Straße 6-14**
**82049 Pullach (DE)**

(54) **VERFAHREN UND ANLAGE ZUR HERSTELLUNG VON DIMETHYLETHER**

(57) Es wird ein Verfahren (100) zur Herstellung von Dimethylether vorgeschlagen, bei dem ein Wasserstoff und Kohlenmonoxid enthaltender Synthesegaseinsatz (A) mit einer Stöchiometriezahl von höchstens 2 bereitgestellt wird, wobei ein erster Anteil (A1) des Synthesegaseinsatzes (A) einer Wassergasshift (10) unterworfen und daran anschließend in einer ersten Waschsektion (21) einer Wascheinheit (20) mit einem physikalischen Waschmittel in Kontakt gebracht und ein zweiter Anteil (A2) des Synthesegaseinsatzes (A), ohne zuvor einer Wassergasshift unterworfen worden zu sein, in einer zweiten Waschsektion (22) der Wascheinheit (20) mit dem physikalischen Waschmittel in Kontakt gebracht wird, wobei das in der ersten Waschsektion (21) verwendete Waschmittel und das in der zweiten Waschsektion (22) der Wascheinheit (20) verwendete Waschmittel in einer gemeinsamen Regenerationssektion (30) der Wascheinheit (20) regeneriert werden, und wobei der in der in der ersten Waschsektion (21) der Wascheinheit (20) gewaschene erste Anteil (A1) des Synthesegaseinsatzes (A) und der in der in der zweiten Waschsektion (21) der Wascheinheit (20) gewaschene zweite Anteil (A2) des Synthesegaseinsatzes (A) jeweils zumindest zum Teil einer gemeinsamen DME-Synthese (50) unterworfen werden. Eine entsprechende Anlage ist ebenfalls Gegenstand der Erfindung.

**Fig. 1**

EP 4 116 285 A1

(52) Gemeinsame Patentklassifikation (CPC): (Forts.)

C-Sets
**C07C 41/01, C07C 43/043;**
**C07C 41/42, C07C 43/043**

**Beschreibung**

Die Erfindung betrifft ein Verfahren und eine Anlage zur Herstellung von Dimethylether gemäß den Oberbegriffen der unabhängigen Patentansprüche.

Hintergrund der Erfindung

Dimethylether (DME) wird derzeit typischerweise durch die Dehydratisierung von Methanol (MeOH) erzeugt. Hierzu wird ein kohlenstoffhaltiger Einsatzstoff, z.B. Erdgas, durch eine geeignete Technologie, z.B. Steam Reforming (SMR), zu einem Synthesegas (Syngas, SG) umgewandelt. Anschließend wird das Synthesegas zu MeOH umgesetzt und dieses dann im nächsten Schritt zu DME dehydratisiert.

Das Synthesegas zur MeOH-Erzeugung zeichnet sich dabei durch eine Stöchiometriezahl (SN, stoichiometric number, stoichiometric module) von etwas mehr als 2 aus. Die Stöchiometriezahl bezeichnet den Quotienten aus der Differenz des Stoffmengenanteils von Wasserstoff ($H_2$) und Kohlendioxid ($CO_2$) und der Summe des Stoffmengenanteils von Kohlenmonoxid (CO) und $CO_2$:

$$SN = (xH_2 - xCO_2)/(xCO + xCO_2)$$

Alternativ kann DME auch in einem Einschrittverfahren erzeugt werden, das auch als Direktsynthese bezeichnet wird. Dazu werden die Katalysatoren zur MeOH- und DME-Synthese in einem Reaktor kombiniert. Auch hier wird Synthesegas aus einem kohlenstoffhaltigen Einsatzstoff (z.B. Erdgas) durch bekannte Technologien hergestellt und anschließend direkt zu DME umgesetzt.

Zu Details sei auf einschlägige Fachliteratur, beispielsweise auf das "DME Handbook" des Japan DME Forum, 2007, oder die Artikel von T. Ogawa et al., "Direct Dimethyl Ether Synthesis", Journal of Natural Gas Chemistry 12, 2003, Seiten 219-227 bzw. S.-H. Lee et al., "Scale Up Study of DME Direct Synthesis Technology", Proceedings of the 24th World Gas Conference, Buenos Aires, 2009, verwiesen.

Je nach Stöchiometriezahl des Synthesegas-Makeups (d.h. des jeweils frisch zur DME-Synthese eingesetzten Synthesegases; der Begriff Makeup wird hier allgemein für eine einen Verbrauch, einen Verlust oder eine Entnahme ausgleichende Einspeisung verwendet) werden bei der DME-Direktsynthese entweder DME und Wasser (bei $SN \geq 2$) oder DME und $CO_2$ (bei $SN \approx 1$) gebildet. Im letzteren Fall wird $CO_2$ i.d.R. mittels einer chemischen Wäsche aus dem Syntheseprodukt abgetrennt und zur Synthesegaserzeugung rezykliert.

Im Falle eines Synthesegaseinsatzes mit SN < 2 muss $CO_2$ aus dem System entfernt werden. Eine Möglichkeit dazu besteht in einem Abzug von $CO_2$ aus einer zur Trennung von DME und $CO_2$ verwendeten Kolonne,

wie in EP 3 521 267 B1 beschrieben. Trennsequenzen für die Auftrennung von Roh-DME, also DME enthaltender Komponentengemische aus einer entsprechenden Synthese, sind ebenfalls in der Patentliteratur beschrieben. Zur Entfernung von DME aus einem Synthesegas-Recycle ist in der EP 3 092 054 B1 auch eine Absorption des Synthesegas-Recycles mit flüssigen $CO_2$ beschrieben.

Vor allem bei einem Synthesegasprozess, der auf einer Vergasung basiert, z.B. von Kohle, Biomasse, Plastik oder flüssigen Einsatzstoffen, werden Synthesegaseinsätze mit einem oftmals unter 1 liegendem Verhältnis von $H_2$ zu CO erzeugt. Um ein für die DME-Synthese geeignetes Verhältnis zu erhalten, kann ein Teil eines derartigen Synthesegaseinsatzes in einer Wassergasshift (WGS) geshiftet werden. Mit einem Bypass um den dabei verwendeten Shiftreaktor kann das gewünschte Verhältnis von $H_2$ zu CO für den nachfolgenden Prozess erhalten werden. Anschließend werden $CO_2$ und andere Sauergase wie Schwefelwasserstoff ($H_2S$) mittels einer Wäsche, beispielsweise einer bekannten Methanolwäsche, entfernt.

Die vorliegende Erfindung stellt sich die Aufgabe, die Integration einer entsprechenden WGS, einer entsprechenden Wäsche, und des nachfolgenden DME-Prozesses zu verbessern.

Offenbarung der Erfindung

Diese Aufgabe wird durch ein Verfahren und eine Anlage zur Herstellung von Dimethylether mit den Merkmalen der unabhängigen Patentansprüche gelöst.

Abhängige Patentansprüche und die nachfolgende Beschreibung betreffen vorteilhafte Ausgestaltungen der Erfindung.

DME kann, wie erwähnt, grundsätzlich über eine Direktsynthese unter Verwendung eines Synthesegases mit einer Stöchiometriezahl SN < 2 erzeugt werden. Die Synthesegaserzeugung erfolgt typischerweise über kohlenstoffhaltige Einsatzstoffe. Nachfolgend soll der Fokus auf einer Synthesegaserzeugung aus Erdgas, Öl, Ölrückständen oder anderen flüssigen Kohlenstoffträgern, insbesondere mittels Partieller Oxidation (POX), oder einer Vergasung von Kohle, Petrolkoks (Petcoke), Biomasse, Plastik oder anderen Reststoffen liegen.

Mit anderen Worten geht die Erfindung von einem Synthesegaseinsatz aus, der durch eine Vergasung oder partielle Oxidation eines festen oder flüssigen kohlenstoffhaltigen Ausgangsstoffs gebildet wird.

Dabei wird der Synthesegaseinsatz konfektioniert, d.h. die Stöchiometriezahl wird über eine WGS, bei der CO mit $H_2O$ in $CO_2$ und $H_2$ umgesetzt wird, in an sich bekannter Weise verändert. Zur Erreichung der für die DME-Direktsynthese benötigten Stöchiometriezahl muss nur ein Teilstrom des Synthesegaseinsatzes über die WGS behandelt werden, während der Rest, wie erwähnt, in einem Bypass um den WGS-Reaktor geführt wird.

Aus dem konfektionierten Synthesegaseinsatz werden anschließend Sauergaskomponenten wie $H_2S$, Carbonylsulfid (COS) und $CO_2$ in einer physikalischen Wäsche, in der typischerweise Methanol (MeOH) als physikalisch wirkendes Waschmittel dient, entfernt, um das Synthesegas für die DME-Direktsynthese zu erhalten.

Ein physikalisch wirkendes Waschmittel zeichnet sich dadurch aus, dass durch dieses Waschmittel ausgewaschene Komponenten im Wesentlichen nicht mit diesem reagieren, also insbesondere keine kovalente Bindung eingehen, sondern die Absorption aufgrund einer rein physikalischen Wechselwirkung erfolgt.

Die Wäsche ist erforderlich, da in der sich anschließenden DME-Synthese $H_2S$ und COS als Katalysatorgifte wirken und der $CO_2$-Gehalt reduziert werden muss, um die Stöchiometriezahl einzustellen und die Langzeitstabilität des Katalysators zu gewährleisten. Das Synthesegas wird herkömmlicherweise als eine Mischung aus geshiftetem und ungeshiftetem Gas in einer Waschkolonne von $H_2S$ und COS (auf einen Wert < 0,1 ppmv) sowie $CO_2$ (auf einen Wert von ca. 0 bis 6 mol%, bevorzugt 2 bis 4 mol%) gereinigt.

Eine Methanolwäsche umfasst typischerweise folgenden Einheiten bzw. Schritte:

1. eine Waschsektion, die bei hohem Druck (ca. 20 bis 80 bar) betrieben wird, und in der der konfektionierte Synthesegaseinsatz mit kaltem MeOH (ca. -30°C bis - 75°C) behandelt wird

2. eine Regenerationssektion zur Regeneration des in der Waschsektion beladenen Waschmittels, bestehend aus den folgenden Hauptprozesseinheiten:

a. Kalte Regeneration: in der die beladenen Waschmittelströme mit Hilfe von Druckreduzierung und gegebenenfalls Stripping mit $CO_2$ und/oder Stickstoff ($N_2$) teilweise regeneriert werden. In diesem Schritt fällt ein sogenanntes Tailgas an, welches im Wesentlichen aus $CO_2$ und $N_2$ besteht. Es kann aber auch ein $CO_2$-Produktstrom gewonnen werden.

b. Heiße Regeneration: hier wird mit Hilfe einer Strippung mit MeOH-Dampf das MeOH weiter aufgereinigt, so dass dieses nach einer entsprechenden Abkühlung und Druckerhöhung mittels einer Pumpe wieder in der Waschsektion eingesetzt werden kann. Eine dabei erhaltene sog. $H_2S$-Fraktion, in der die Schwefelkomponenten (insbesondere $H_2S$ und COS) in konzentrierter Form (typischerweise mit > 25 mol%) vorliegen, kann als Einsatzstoff zur Gewinnung von elementarem Schwefel nach dem Claus-Prozess verwendet werden.

c. Trennung von MeOH und $H_2O$: Überschüssiges $H_2O$ wird aus dem Waschmittelkreislauf entfernt.

Ist hier vereinfacht von einer Methanolwäsche oder auch allgemeiner von einer Wäsche die Rede, soll dieser Begriff für eine physikalische Wäsche mit einem organischen Waschmittel stehen, das insbesondere aus MeOH besteht oder dieses enthält, und das bei einer Temperatur von weniger als -10 °C, insbesondere weniger als -20 °C oder weniger als -30 °C und insbesondere mehr als -100 °C, insbesondere mehr als -80 °C, insbesondere mehr als -75 °C, und auf einem Druck von mehr als 10 bar, insbesondere mehr als 20 bar, und insbesondere bis zu 100 bar, insbesondere bis zu 80 bar, in Kontakt mit einem ein oder mehrere Sauergase, insbesondere $CO_2$ und $H_2S$, enthaltenden Gasgemisch gebracht wird. Insbesondere wird dieses Gasgemisch dabei an dem einen oder den mehreren Sauergasen abgereichert und das Waschmittel dagegen unter Erhalt eines beladenen Waschmittels entsprechend angereichert. Unter Abreicherung des beladenen Waschmittels an dem einen oder den mehreren Sauergasen wird das Waschmittel für einen erneuten Einsatz regeneriert.

Der auf diese Weise behandelte konfektionierte Synthesegaseinsatz wird in die DME-Synthese mit anschließender Trennsequenz weitergeführt.

Die Erfindung schlägt nun, abweichend zum Bekannten, eine weitergehende Integration der beiden Anlagenteile physikalische Wäsche (insbesondere Methanolwäsche) und DME-Synthese vor. Gemäß einem Hauptaspekt der Erfindung werden der geshiftete und der ungeshiftete Teil des Synthesegaseinsatzes dabei in zwei unterschiedlichen, über eine gemeinsam nutzbare Regenerationssektion verbundenen Waschsektionen behandelt. Dies bedeutet zwar einen höheren apparativen Aufwand, jedoch sinkt der Einsatz an Betriebsmitteln sowie der Waschmittelumlauf, so dass die Gesamtkostenbilanz des erfindungsgemäßen Verfahrens positiv ausfällt.

Mit anderen Worten schlägt die Erfindung ein Verfahren zur Herstellung von Dimethylether vor, bei dem ein Wasserstoff und Kohlenmonoxid enthaltender Synthesegaseinsatz mit einer Stöchiometriezahl von höchstens 2, insbesondere 1,0 bis 1,8, weiter insbesondere 1,1 bis 1,4, bereitgestellt wird, wobei ein erster Teil eines Wasserstoff und Kohlenmonoxid enthaltenden Synthesegaseinsatzes einer Wassergasshift unterworfen und daran anschließend in einer ersten Waschsektion einer Wascheinheit mit einem physikalischen Waschmittel in Kontakt gebracht wird und ein zweiter Teil des Synthesegaseinsatzes, ohne zuvor einer Wassergasshift unterworfen worden zu sein, in einer zweiten Waschsektion derselben Wascheinheit mit dem physikalischen Waschmittel in Kontakt gebracht wird.

Das in der ersten Waschsektion verwendete Waschmittel und das in der zweiten Waschsektion der Wascheinheit verwendete Waschmittel werden in einer gemeinsamen Regenerationssektion der Wascheinheit regeneriert, und der in der in der ersten Waschsektion der Wascheinheit gewaschene geshiftete erste Teil des Synthesegaseinsatzes und der in der in der zweiten Waschsektion der Wascheinheit gewaschene zweite Teil des Synthesegaseinsatzes werden jeweils zumindest zum Teil einer gemeinsamen DME-Synthese unterworfen.

Die physikalische Wäsche kann insbesondere als eine erläuterte Methanolwäsche ausgebildet sein, so dass die oben gemachten Ausführungen zu Waschmitteln und Waschbedingungen auch für die in der Erfindung eingesetzte Wäsche gelten.

Mit anderen Worten kann das in der ersten Waschsektion der Wascheinheit und das in der zweiten Waschsektion der Wascheinheit verwendete Waschmittel Methanol sein oder umfassen, wobei das Inkontaktbringen des geshifteten ersten Teils des Synthesegaseinsatzes in der ersten Waschsektion der Wascheinheit mit dem Waschmittel und das Inkontaktbringen des zweiten Teils des Synthesegaseinsatzes in der zweiten Waschsektion der Wascheinheit mit dem Waschmittel bei einer Temperatur von weniger als -20 °C und einem Druck von mehr als 20 bar erfolgt. Zu weiteren Temperatur- und Druckbereichen sei auf die obigen Ausführungen verwiesen.

Insbesondere kann in der gemeinsamen Regenerationssektion der im Rahmen der vorliegenden Erfindung eingesetzten Wascheinheit zur Regeneration des in der ersten Waschsektion der Wascheinheit verwendeten Waschmittels und des in der zweiten Waschsektion der Wascheinheit verwendeten Waschmittels eine Heißregeneration und eine Kaltregeneration sowie eine MeOH/$H_2$O-Trennung vorgenommen werden. Mittels der MeOH/$H_2$O-Trennung in der Regenerationssektion bereitgestelltes MeOH kann zumindest zum Teil der DME-Synthese zugeführt werden.

Im Rahmen der vorliegenden Erfindung wird ein mittels der DME-Synthese bereitgestelltes Produktgemisch zumindest zum Teil einer DME-Trennung unterworfen. Mittels der DME-Trennung können dabei ein Strom nicht umgesetzten Synthesegases, der insbesondere einer dritten Waschsektion der Wascheinheit zugeführt werden kann, und/oder ein MeOH/$H_2$O-Strom, der insbesondere der MeOH/$H_2$O-Trennung in der Regenerationssektion der Wascheinheit zugeführt werden kann, und/oder ein $CO_2$-Strom, der insbesondere aus dem Verfahren ausgeleitet werden kann, und/oder ein DME-Strom (Produktstrom), der ebenfalls insbesondere aus dem Verfahren ausgeleitet werden kann, bereitgestellt werden.

Die DME-Trennung kann insbesondere als Trennsequenz mit unterschiedlichen Trenneinheiten ausgebildet sein. Der Purgestrom aus der DME-Trennsequenz, nämlich der erwähnte Strom nicht umgesetzten Synthesegases, wird herkömmlicherweise als Brenngas verwendet, da dieser zwar reich an CO und $H_2$ ist, aber auch bedeutende Mengen an $CO_2$ aufweist. Dieser Purgestrom kann im Rahmen der vorliegenden Erfindung

zur $CO_2$-Entfernung, wie erwähnt, in einer dritten Waschsektion der zur Aufbereitung des Synthesegaseinsatzes verwendeten Wascheinheit gewaschen werden, wobei als Waschmittel eingesetztes Methanol in der gemeinsamen Regenerationssektion regeneriert wird. Durch die $CO_2$-Entfernung wird die Qualität des Purgestromes erhöht und dieser kann daher beispielsweise zur Wasserstoffgewinnung in einer PSA-Anlage und/oder in einer kryogenen CO-Gewinnung (CCSU) genutzt werden.

Sowohl die DME-Trennsequenz als auch die Wascheinheit benötigen eine MeOH/$H_2$O-Trennung, welche, wie bereits angesprochen, in einer Ausgestaltung der Erfindung kombiniert werden können, so dass anstatt zweier Trennkolonnen mit Reboiler nur noch eine benötigt wird. Es ist auch möglich, eine kombinierte DME/MeOH/$H_2$O-Trennung unter Verwendung einer Trennwandkolonne durchzuführen.

Sogenanntes Flashgas und an $CO_2$ reiches Gas aus einer in der DME-Trennung zur Trennung von $CO_2$ und DME eingesetzten $CO_2$/DME-Kolonne können kombiniert und mit einem Kompressor zur Wascheinheit rezykliert werden. Dies erhöht die Synthesegasausbeute sowohl der DME-Synthese als auch der Wascheinheit. Der Druck der DME/$CO_2$-Kolonne kann entsprechend angepasst werden.

Ein Synthesegasrecycle kann im Rahmen der vorliegenden Erfindung, anstatt wie in bekannten Verfahren mit einem DME-Rücklauf in seinem $CO_2$-Gehalt reduziert zu werden, in die Wascheinheit geführt werden. Hierbei ist ein Verdichter für den kalten Synthesegasrecycle zu installieren, um die Druckniveaus des Synthesegaseinsatzes aus der Vergasung bzw. des Einsatzes und des Synthesegasrecycles anzugleichen. Ein Teil des Synthesegasrecycles kann an der Wascheinheit vorbeigeführt werden, um den $CO_2$-Anteil im Synthesegas für die DME-Synthese einzustellen.

Ein MeOH-Makeup, der für die Wascheinheit notwendig ist, kann im Rahmen der vorliegenden Erfindung aus dem DME-Prozess stammen. In der DME-Synthese entstehen auch Spuren an höheren Alkoholen, z.B. Ethanol oder Propanol, sowie Acetaldehyd, die sich im MeOH befinden. Normalerweise muss ein Teil dieses MeOH-Stroms verworfen werden, um die Rückführung der höheren Alkohole in den DME-Reaktor zu reduzieren (da diese zu weiteren Nebenreaktionen führen würden). Wird dieser Purge-Strom als MeOH-Makeup in der Wascheinheit genutzt, beeinflusst dies die Wäsche nicht und ein entsprechender Strom geht nicht verloren. Diese Spuren reichern sich in der Wascheinheit an und können ohne negative Einflüsse auf die Performance bei einer höheren Konzentration und damit effizienter aus dem Prozess entnommen werden.

Mit anderen Worten wird in einer Ausgestaltung der vorliegenden Erfindung Methanol, das in der DME-Synthese als Nebenprodukt anfällt, zum Ausgleich eines Waschmittelverlusts in die Wascheinheit geführt.

Ein Aspekt der Erfindung besteht in der Nutzung einer gemeinsamen Kälteanlage, die Kälte für die DME-Trennsequenz und für die Wascheinheit. Vorteilhafterweise können also die Wascheinheit und die DME-Trennung unter Verwendung von Kältemittel betrieben werden, welches mittels einer gemeinsamen Kältemitteleinheit bereitgestellt bzw. temperiert wird. Dabei kann auch insbesondere eine Anpassung der Kälteniveaus aneinander erfolgen, also insbesondere in der Wascheinheit bzw. deren Waschsektionen ein Waschmitteltemperaturbereich verwendet werden, der unter Verwendung des Kältemittels in einem ersten Kältemitteltemperaturbereich erhalten wird, und in der DME-Trennung kann eine Trenneinheit in einem Trenntemperaturbereich betrieben werden, der unter Verwendung des Kältemittels in einem zweiten Kältemitteltemperaturbereich erhalten wird. Der erste und zweite Kältemitteltemperaturbereich gleichen dabei einander, überschneiden sich oder liegen um nicht mehr als 10 K auseinander. Es kann insbesondere auch eine Nutzung von kaltem MeOH aus der Wascheinheit zur Abkühlung in der Trennsequenz der DME-Anlage erfolgen.

Zur Reduktion der Anzahl an Verdichtern kann im Rahmen der vorliegenden Erfindung in Ausgestaltungen vorgesehen sein, dass

- der Synthesegaseinsatz dem Verfahren in einem Druckbereich von 30 bis 40 bar zugeführt, ein Verdichter stromab der WGS des ersten Teils des Synthesegaseinsatzes bereitgestellt wird und zumindest die erste und zweite Waschsektion der Wascheinheit in einem Druckbereich oberhalb der DME-Synthese betrieben werden, wobei die DME-Synthese insbesondere in einem Druckbereich von 50 bis 70 bar betrieben wird, oder

- der Synthesegaseinsatz dem Verfahren in einem Druckbereich von 40 bis 57 bar zugeführt und ein Verdichter stromab der Wascheinheit bzw. zumindest stromab von deren erster und zweiter Waschsektion bereitgestellt wird (wodurch die Wascheinheit zwar auf einem niedrigen Druck betrieben wird, aber der Verdichter kleiner ausfällt), oder

- der Synthesegaseinsatz dem Verfahren in einem Druckbereich von über 57 zugeführt und im Verfahren nicht weiter verdichtet wird.

Zu Merkmalen und Vorteilen der erfindungsgemäß vorgeschlagenen Anlage und vorteilhafter Ausgestaltungen hiervon sei auf den entsprechenden unabhängigen Patentanspruch und ferner die obigen Ausführungen bezüglich des erfindungsgemäßen Verfahrens und vorteilhafter Ausgestaltungen hiervon ausdrücklich verwiesen. Insbesondere ist die erfindungsgemäß vorgeschlagene Anlage bzw. sind entsprechende Ausgestaltungen hiervon dazu eingerichtet, entsprechende Verfahrensvarianten durchzuführen.

Die erfindungsgemäß vorgeschlagenen Maßnahmen, die in dem beanspruchten Verfahren und der beanspruchten Anlage sowie in ihren jeweiligen Ausgestaltungen ihren Niederschlag finden, und die die Kombination einer Shifteinheit, einer Wascheinheit, die insbesondere zur Durchführung einer Methanolwäsche eingerichtet ist, und einer DME-Synthese umfasst, bieten, zusammengefasst und teils die obigen Ausführungen wiederholend, folgende Vorteile:

- Die Methanolwäsche ist in der Lage, $H_2S$ und COS abzutrennen, so dass auf eine Hydrolyse zur Umsetzung von COS zu $H_2S$, wie sei beispielsweise beim Einsatz einer Aminwäsche erforderlich ist, verzichtet werden kann.

- Der restliche $CO_2$-Gehalt im Synthesegas zur DME-Synthese kann je nach Optimierung der DME-Synthese eingestellt werden (je nach Bedarf von 10 ppmv bis in den Prozentbereich).

- Das Synthesegas aus der erfindungsgemäß durchgeführten physikalischen Wäsche ist insbesondere frei von anderen Spurenkomponenten wie beispielsweise Blausäure (HCN), Ammoniak (NH3), Benzol/Toluol/Xylol (BTX) und Mercaptanen.

- Das Synthesegas aus der Wäsche ist trocken.

- Das Waschmittel der Wäsche ist insbesondere Methanol, so dass Spuren davon im Syngas zur DME-Synthese nicht schädlich sind.

- Der Synthesegasverbrauch wirkt gesenkt.

- Die Investitionskosten werden gesenkt.

Eine Ausgestaltung der Erfindung wird nachfolgend unter Bezugnahme auf die beigefügte Zeichnung weiter erläutert.

Kurze Beschreibung der Zeichnung

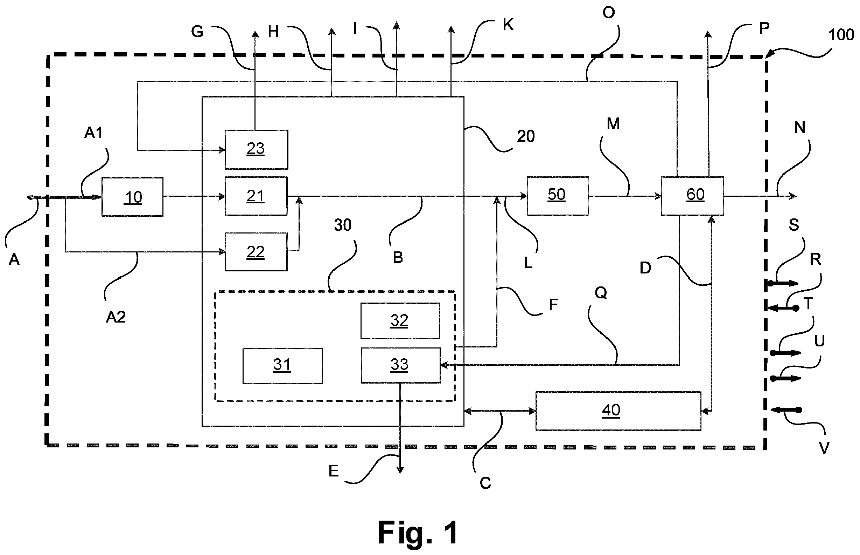

Figur 1 veranschaulicht ein Verfahren gemäß einer Ausgestaltung der Erfindung.

Ausführliche Beschreibung der Zeichnung

In Figur 1 ist ein Verfahren gemäß einer Ausführungsform der Erfindung veranschaulicht und insgesamt mit 100 bezeichnet. Wird nachfolgend auf Aspekte eines derartigen Verfahrens 100 oder ein derartiges Verfahren 100 insgesamt Bezug genommen, so gelten die entsprechenden Erläuterungen auch für eine Anlage gemäß einer Ausgestaltung der Erfindung und Aspekten hiervon.

Dem Verfahren 100 wird ein Synthesegaseinsatz, beispielsweise aus einem erläuterten Vergasungsprozess oder einer partiellen Oxidation, in Form eines Einsatzstroms A zugeführt, welcher insbesondere eine Stöchiometriezahl von weniger als 2 aufweist. Ein erster Anteil A1 des Einsatzstroms A wird in einer Wassergasshift 10 bearbeitet, um die Stöchiometriezahl, wie eingangs erläutert, einzustellen. Ein zweiter Anteil A2 wird nicht entsprechend behandelt.

Eine Wascheinheit 20, die eine erste Waschsektion 21, eine zweite Waschsektion 22 und eine dritte Waschsektion 23 sowie eine Regenerationssektion 30 aufweist, ist bereitgestellt. Der (geshiftete) erste Anteil A1 des Einsatzstroms A wird dabei der ersten Waschsektion 21 zugeführt und in dieser mit einem physikalischen Waschmittel, insbesondere kaltem Methanol bei hohem Druck, in Kontakt gebracht. Dagegen wird der (ungeshiftete) zweite Anteil A2 des Einsatzstroms A der zweiten Waschsektion 22 zugeführt und in dieser mit dem physikalischen Waschmittel, insbesondere kaltem Methanol bei hohem Druck, in Kontakt gebracht.

Die in den Waschsektionen 21, 22 an $CO_2$ und anderen Sauergasen, falls vorhanden, abgereicherten Anteile A1, A2 des Einsatzstroms A werden stromab der Waschsektionen 21, 22 zu einem Sammelstrom B vereinigt.

In der Regenerationssektion 30 erfolgt eine Regeneration des beladenen Waschmittels in einer an sich bekannten Weise, jedoch gemeinsam für die erste und zweite Waschsektion 21, 22. Die Regenerationssektion 30 umfasst dabei eine Kaltregeneration 31 und eine Heißregeneration 32, wie eingangs erläutert, sowie eine MeOH/$H_2O$-Trennung 33. Mit der Regenerationssektion 30 kann in bekannter Weise eine Kälteeinheit 40 assoziiert sein, die Kältemittelströme C und D liefern kann, unter anderem um die Kaltregeneration 31 zu betreiben. In der Regenerationssektion 30 kann ein Abwasserstrom E anfallen, der aus dem Verfahren 100 ausgeleitet werden kann, sowie ein Methanolstrom F.

Aus der Wascheinheit 20 bzw. der Regenerationssektion 30 können ein Strom G von gereinigtem Synthesegas, ein $CO_2$-Strom H mittlerer Reinheit, ein Tailgasstrom I und eine $H_2S$-Fraktion K ausgeleitet werden.

Nach optionaler Zugabe des Methanolstroms F oder eines Teils hiervon zu dem Sammelstrom B, wird dieser, nun mit L bezeichnet, als Synthesegas einer DME-Synthese 50 zugeführt, die als Direktsynthese betrieben werden kann. Ein Produktstrom M der DME-Sytnthese wird einer DME-Trennung 60 zugeführt, in der ein DME-Strom N, ein Strom O aus unkonvertiertem Synthesegas, ein $CO_2$-Strom P hoher Reinheit und ein MeOH/$H_2O$-Strom Q gebildet werden können. Die DME-Trennung 60 kann mit dem bereits erwähnten Kältemittelstrom D aus der Kälteeinheit 40 betrieben werden.

Der Strom O aus unkonvertiertem Synthesegas kann in der dritten Waschsektion 23 der Wascheinheit 20 zu dem bereits erwähnten Strom G von gereinigtem Synthesegas aufbereitet werden. Der MeOH/$H_2O$-Strom Q kann dagegen der MeOH/$H_2O$-Trennung 33 der Regenerationssektion 30 der Wascheinheit 20 zugeführt werden.

Dem Verfahren 100 insgesamt wird ein Kühlwasserstrom R zugeführt und aus dem Verfahren 100 insgesamt wird ein Kühlwasserstrom S ausgeführt. Exportströme sind ferner ein Niederdruck-$N_2$-Strom T und ein Dampfstrom U. Das Verfahren 100 wird mit elektrischer Energie V betrieben.

## Patentansprüche

1. Verfahren (100) zur Herstellung von Dimethylether, bei dem ein Wasserstoff und Kohlenmonoxid enthaltender Synthesegaseinsatz (A) mit einer Stöchiometriezahl von höchstens 2 bereitgestellt wird, wobei ein erster Anteil (A1) des Synthesegaseinsatzes (A) einer Wassergasshift (10) unterworfen und daran anschließend in einer ersten Waschsektion (21) einer Wascheinheit (20) mit einem physikalischen Waschmittel in Kontakt gebracht und ein zweiter Anteil (A2) des Synthesegaseinsatzes (A), ohne zuvor einer Wassergasshift unterworfen worden zu sein, in einer zweiten Waschsektion (22) der Wascheinheit (20) mit dem physikalischen Waschmittel in Kontakt gebracht wird, wobei das in der ersten Waschsektion (21) verwendete Waschmittel und das in der zweiten Waschsektion (22) der Wascheinheit (20) verwendete Waschmittel in einer gemeinsamen Regenerationssektion (30) der Wascheinheit (20) regeneriert werden, und wobei der in der in der ersten Waschsektion (21) der Wascheinheit (20) gewaschene erste Anteil (A1) des Synthesegaseinsatzes (A) und der in der in der zweiten Waschsektion (21) der Wascheinheit (20) gewaschene zweite Anteil (A2) des Synthesegaseinsatzes (A) jeweils zumindest zum Teil einer gemeinsamen DME-Synthese (50) unterworfen werden.

2. Verfahren (100) nach Anspruch 1, bei dem das in der ersten Waschsektion (21) der Wascheinheit (20) verwendete Waschmittel und das in der zweiten Waschsektion (22) der Wascheinheit (20) verwendete Waschmittel Methanol ist oder umfasst, wobei das Inkontaktbringen des ersten Anteils (A1) des Synthesegaseinsatzes (A) in der ersten Waschsektion (21) der Wascheinheit (20) mit dem Waschmittel und das Inkontaktbringen des zweiten Anteils (A1) des Synthesegaseinsatzes (A) in der zweiten Waschsektion (22) der Wascheinheit (20) mit dem Waschmittel bei einer Temperatur von weniger als -20 °C und einem Druck von mehr als 20 bar erfolgt.

3. Verfahren (100) nach Anspruch 1 oder nach Anspruch 2, bei dem in der gemeinsamen Regenerationssektion (30) der Wascheinheit (20) zur Regeneration des in der ersten Waschsektion (21) verwendeten Waschmittels und des in der zweiten Waschsektion (22) der Wascheinheit (20) verwendeten Waschmittels eine Heißregeneration (31) und eine Kaltregeneration (32) sowie eine MeOH/$H_2O$-Tren-

nung (33) vorgenommen wird.

4. Verfahren (100) nach Anspruch 3, bei dem mittels der MeOH/$H_2$O-Trennung (33) bereitgestelltes MeOH zumindest zum Teil der DME-Synthese (50) zugeführt wird.

5. Verfahren (100) nach Anspruch 3 oder nach Anspruch 4, bei dem ein mittels der DME-Synthese (50) bereitgestelltes Produktgemisch (M) zumindest zum Teil einer DME-Trennung (60) unterworfen wird.

6. Verfahren (100) nach Anspruch 5, bei dem mittels der DME-Trennung (60) ein Strom (Q) bei der DME-Synthese nicht umgesetzten Synthesegases, der insbesondere einer dritten Waschsektion (23) der Wascheinheit (20) zugeführt wird, und/oder ein MeOH/$H_2$O-Strom (Q), der insbesondere der MeOH/$H_2$O-Trennung (33) in der Regenerationssektion (30) der Wascheinheit (20) zugeführt wird, und/oder ein $CO_2$-Strom (P), der insbesondere aus dem Verfahren (100) ausgeleitet wird, und/oder ein DME-Strom (N), der insbesondere aus dem Verfahren (100) ausgeleitet wird, bereitgestellt werden.

7. Verfahren (100) nach einem der Ansprüche 4 bis 6, bei dem Methanol, das in der DME-Synthese (30) als Nebenprodukt anfällt, zum Ausgleich eines Waschmittelverlusts in die Wascheinheit (20) geführt wird.

8. Verfahren (100) nach einem der Ansprüche 4 bis 7, bei dem die Wascheinheit (20) und die DME-Trennung unter Verwendung von Kältemittel betrieben werden, das mittels einer gemeinsamen Kältemitteleinheit (40) bereitgestellt wird.

9. Verfahren (100) nach Anspruch 8, bei dem in der Wascheinheit (10) ein Waschmitteltemperaturbereich verwendet wird, der unter Verwendung des Kältemittels in einem ersten Kältemitteltemperaturbereich erhalten wird, und bei dem in der DME-Trennung (60) eine Trenneinheit in einem Trenntemperaturbereich betrieben wird, der unter Verwendung des Kältemittels in einem zweiten Kältemitteltemperaturbereich erhalten wird, wobei der erste und zweite Kältemitteltemperaturbereich einander gleichen, sich diese überschneiden, oder diese um nicht mehr als 10 K auseinanderliegen.

10. Verfahren (100) nach einem der vorstehenden Ansprüche, bei dem der Synthesegaseinsatz (A) dem Verfahren (100) in einem Druckbereich von 30 bis 40 bar zugeführt wird, wobei ein Verdichter stromab der Wassergasshift (10) des ersten Anteils (A1) bereitgestellt wird und die Wascheinheit (20) in einem Druckbereich oberhalb der DME-Synthese (50) betrieben wird.

11. Verfahren (100) nach einem der Ansprüche 1 bis 9, bei dem der Synthesegaseinsatz (A) dem Verfahren in einem Druckbereich von 40 bis 57 bar zugeführt wird, wobei ein Verdichter stromab der Wascheinheit (20) bereitgestellt wird.

12. Verfahren (100) nach einem der Ansprüche 1 bis 9, bei dem der Synthesegasensatz (A) dem Verfahren (100) in einem Druckbereich von mehr als 57 bar zugeführt wird und in dem Verfahren (100) nicht weiter verdichtet wird.

13. Anlage zur Herstellung von Dimethylether, die dafür eingerichtet ist, einen Wasserstoff und Kohlenmonoxid enthaltender Synthesegaseinsatz (A) mit einer Stöchiometriezahl von höchstens 2 bereitzustellen, einen ersten Anteil (A1) des Synthesegaseinsatzes (A) einer Wassergasshift (10) zu unterwerfen und daran anschließend in einer ersten Waschsektion (21) einer Wascheinheit (20) mit einem physikalischen Waschmittel in Kontakt zu bringen, einen zweiten Anteil (A2) des Synthesegaseinsatzes (A), ohne diesen zuvor der Wassergasshift unterworfen zu haben, in einer zweiten Waschsektion (22) der Wascheinheit (20) mit dem physikalischen Waschmittel in Kontakt zu bringen, das in der ersten Waschsektion (21) verwendete Waschmittel und das in der zweiten Waschsektion (22) der Wascheinheit (20) verwendete Waschmittel in einer gemeinsamen Regenerationssektion (30) der Wascheinheit (20) zu regenerieren, und den in der ersten Waschsektion (21) der Wascheinheit (20) gewaschenen ersten Anteil (A1) des Synthesegaseinsatzes (A) und den in der in der zweiten Waschsektion (21) der Wascheinheit (20) gewaschenen zweiten Anteil (A2) des Synthesegaseinsatzes (A) jeweils zumindest zum Teil einer gemeinsamen DME-Synthese (50) zu unterwerfen.

Fig. 1

Europäisches
Patentamt
European
Patent Office
Office européen
des brevets

**EUROPÄISCHER RECHERCHENBERICHT**

Nummer der Anmeldung

EP 21 02 0361

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/126259 A1 (DEN BERG ROBERT VAN [NL] ET AL) 21. Mai 2009 (2009-05-21) <br> * Absätze [0030], [0034], [0035] * <br> * Absätze [0039], [0044] * <br> * Absätze [0045], [0046] * <br> * Absätze [0080] - [0085]; Abbildung 1 * <br> ----- | 1-13 | INV. <br> C07C41/01 <br> C07C41/38 <br> C07C43/04 <br> C07C41/42 <br> C01B3/48 |
| A | EP 2 898 943 A1 (LINDE AG [DE]; TECH UNIVERSITÄT MÜNCHEN [DE]) 29. Juli 2015 (2015-07-29) <br> * Absätze [0020] - [0034] * <br> ----- | 1 | |

**RECHERCHIERTE SACHGEBIETE (IPC)**

C07C
C01B

Der vorliegende Recherchenbericht wurde für alle Patentansprüche erstellt

| Recherchenort <br> **Den Haag** | Abschlußdatum der Recherche <br> **22. Dezember 2021** | Prüfer <br> **Kardinal, Siegmar** |
|---|---|---|

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 02 0361

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-12-2021

| Im Recherchenbericht angeführtes Patentdokument | Datum der Veröffentlichung | Mitglied(er) der Patentfamilie | Datum der Veröffentlichung |
|---|---|---|---|
| US 2009126259 A1 | 21-05-2009 | AU 2009331847 A1 | 07-07-2011 |
| | | CN 102264679 A | 30-11-2011 |
| | | EP 2373602 A1 | 12-10-2011 |
| | | US 2009126259 A1 | 21-05-2009 |
| | | WO 2010072424 A1 | 01-07-2010 |
| | | ZA 201104258 B | 29-02-2012 |
| EP 2898943 A1 | 29-07-2015 | AU 2015213150 A1 | 21-07-2016 |
| | | BR 112016015885 B1 | 01-12-2020 |
| | | CA 2936279 A1 | 06-08-2015 |
| | | CN 105980341 A | 28-09-2016 |
| | | EP 2898943 A1 | 29-07-2015 |
| | | JP 2017508732 A | 30-03-2017 |
| | | KR 20160113649 A | 30-09-2016 |
| | | MX 366823 B | 25-07-2019 |
| | | RU 2016134895 A | 05-03-2018 |
| | | US 2016326078 A1 | 10-11-2016 |
| | | WO 2015113727 A1 | 06-08-2015 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3521267 B1

- EP 3092054 B1

**In der Beschreibung aufgeführte Nicht-Patentliteratur**

- **T. OGAWA et al.** Direct Dimethyl Ether Synthesis. *Journal of Natural Gas Chemistry,* 2003, vol. 12, 219-227

- Scale Up Study of DME Direct Synthesis Technology. **S.-H. LEE et al.** Proceedings of the 24th World Gas Conference. Buenos Aires, 2009